## Europäisches Patentamt
## European Patent Office
## Office européen des brevets

(19)

(11) Veröffentlichungsnummer: **0 021 301**
**A2**

(12) # EUROPÄISCHE PATENTANMELDUNG

(21) Anmeldenummer: 80103340.8

(22) Anmeldetag: 14.06.80

(51) Int. Cl.³: **A 61 M 1/03**

(30) Priorität: 22.06.79 DE 2925143

(43) Veröffentlichungstag der Anmeldung: 07.01.81
Patentblatt 81/1

(84) Benannte Vertragsstaaten: DE FR GB IT SE

(71) Anmelder: Biotest-Serum-Institut GmbH,
Flughafenstrasse 4, D-6000 Frankfurt-Niederrad (DE)

(72) Erfinder: Handel, Klaus-Dieter, Dr. Dipl. Chem.,
Pallaswiesenstrasse 14, D-6100 Darmstadt (DE)
Erfinder: Bonhard, Klaus, Dr. Dipl. chem.,
Sandeldamm 16, D-6450 Hanau (DE)

(74) Vertreter: Beil, Walter, Dr. et al, BEIL, WOLFF & BEIL
Rechtsanwälte Adelonstrasse 58, D-6230 Frankfurt am
Main 80 (DE)

(54) Vorrichtung für die kontinuierliche Plasmapherese.

(57) Vorrichtung für die kontinuierliche Plasmapherese. Diese Vorrichtung besteht aus Blutseite, bestehend aus runder Platte (1) mit Blutzuflusskanal (3), Blutabflusskanal (4), Kanälen (5), die Blutzuflusskanal (3) und Blutabflusskanal (4) verbinden, und Kanalabgrenzungen (6), wobei Breite der Kanäle das 4- bis 20fache deren Tiefe beträgt, und der Blutseite gegenüberliegender Plasmaseite, bestehend aus runder Platte (2) mit Plasmaabflusskanälen (4a) und/ oder (4b), wobei die Dimensionen von Platte (2) und der Plasmaabflusskanäle (4a) bzw. (4b) den Dimensionen der Platte (1) und dem Blutzuflusskanal (3) und dem Blutabflusskanal (4) entsprechen, den Kanälen (5a) die den Plasmaabflusskanal (4a) und den Plasmaabflusskanal (4b) verbinden, und den Kanalbegrenzungen (6a), die nicht deckungsgleich mit Kanalmuster der Blutseite und so gestaltet sind, dass sie die dazwischenliegende Membran abzustützen vermögen, und dass durch Zusammenbau der beiden Platten eine Filtrationszone gebildet wird, wobei Kanäle (5) über die Filtrationszone hinausreichen.

Die Kapazität derselben gestattet Anwendung bei Plasmaspende und ist trotzdem von Handhabung und wirtschaftlichem Standpunkt her realisierbar.

0021301

Unsere Nr. 22 923                          Pr/br

Biotest-Serum-Institut GmbH

Flughafenstraße 4

6000 Frankfurt-Niederrad

Vorrichtung für die kontinuierliche Plasmapherese

Die Erfindung betrifft eine Vorrichtung zur kontinuierlichen Plasmapherese.

Unter Plasmapherese versteht man die Auftrennung von Vollblut in eine Plasmafraktion und eine Zellenfraktion.

Bei der Blutentnahme zum Zwecke der Plasmaspende direkt vom Spender ist es wünschenswert, die Zellkomponenten wieder an denselben zurückzuführen, da sich der Spender bei dieser Rückführung wesentlich schneller erholt, als bei Nichtrückführung. Dadurch kann in kürzeren Abständen Plasma gespendet werden.
Die Fähigkeit eines Spenders, in verhältnismäßig kurzen Zeitabständen Plasma spenden zu können, ist besonders in den Fällen von Bedeutung, in denen der Spender eine seltene Blutgruppenkonstellation oder eine besondere Plasmazusammensetzung aufweist.

0021301

Im allgemeinen erfolgte die Plasmapherese bisher dadurch, daß das dem Spender entnommene Vollblut in ein stabilisatorhaltiges Beutelsystem gefüllt wurde, in diesem Beutel die zellulären und plasmatischen Bestandteile durch Zentrifugieren getrennt und die zellulären Anteile retransfundiert wurden.

Ein solches System bezeichnet man als diskontinuierliche Plasmapherese.

Bei einer derartigen diskontinuierlichen Plasmapherese wird im Laufe des Verfahrens der Beutel vom Spender getrennt und einer Vielzahl von Behandlungsschritten unterworfen, so daß die Gefahr einer Kontamination und einer Verwechselung der Beutel mit Beutel anderer gleichzeitig an einer Plasmapherese teilnehmenden Spendern bei der Retransfusion der Blutzellen nicht auszuschließen sind.

Bei einer Spende können bis zu 500 ml Plasma gespendet werden, wozu 1000 ml Blut benötigt werden. Diese dürfen aber nicht auf einmal entnommen werden, um den Spender nicht zu stark zu belasten, so daß dieses Verfahren in zwei Schritten durchgeführt werden muß.

Man war deshalb bestrebt, ein System bzw. eine Vorrichtung zu finden, mit deren Hilfe ein direkter, kontinuierlicher Kreislauf - Blutentnahme beim Spender, Auftrennung in Plasma- und Zellfraktion und Rückführung der Zellfraktion zum Spender - ermöglicht wird.

In der DE-PS 21 00 209 wird eine Vorrichtung zur Abtrennung von Blutplasma von anderen Blutkomponenten durch Filtration durch eine mikroporöse Membran beschrieben. In dieser Vorrichtung wird Vollblut parallel zur Oberfläche der Membran mit einem bestimmten Druckgefälle und einer bestimmten Strömungsgeschwindigkeit geleitet. Zellfreies Plasma wird als Filtrat von der Plasmaseite der Membran, und die zelluläre Fraktion wird am Auslaßende des Blutkanals gewonnen. Die DE-PS beschreibt, daß mit Hilfe dieser Vorrichtung aus einer 10 ml-Probe frischen Blutes in einer Filtrierzeit von 15 bis 20 Minuten etwa 3,0 bis 3,4 ml Plasma gewonnen werden können. Obgleich mit dieser Vorrichtung eine kontinuierliche Plasmapherese durchführbar ist, reicht die damit erzielbare Filterkapazität nur für relativ kleine Plasmamengen für Analysenzwecke aus, jedoch nicht für die praktische Anwendung zur Gewinnung von 500 ml zellfreien Plasmas in nicht länger als 60 bis 90 Minuten, was von einer normalen Plasmapheresetechnik verlangt wird.

Gemäß eines in Abstracts (II) des XVII Congress of the International Society of Hematology, XV Congress of the International Society of Blood Transfusion vom 27. und 28. Juli 1978 beschriebenen Modells wird die Kapazität einer Vorrichtung zur kontinuierlichen Plasmapherese dadurch ermöglicht, daß deren Strömungskanäle, deren eine Wand jeweils aus einer mikroporösen Membran gebildet wird, vom Bluteinlaß zum Blutauslaß hin entlang ihrer Länge divergieren, wodurch die Schergeschwindigkeiten an der Grenzfläche Blut/Membran sich entlang der Strömungsbahn von einem Maximalwert am Einlaßende zu einem Minimalwert am Auslaßende verändern. Diese Veränderung der Schergeschwindigkeit entspricht der Veränderung des Transmembrandruckes entlang der Kanäle. Der Transmembrandruck darf bei einer bestimmten Schergeschwindigkeit einen bestimmten Wert nicht überschreiten, damit keine Hämolyse eintritt. Mit Hilfe dieses Gerätes kann die Schergeschwindigkeit gesteuert und somit eine optimale Filtrationsgeschwindigkeit ohne Hämolyse sichergestellt werden.

Die Bedingungen, die an eine kontinuierliche Plasmapherese gestellt werden, werden mit dieser Vorrichtung zwar erfüllt, jedoch ist sie in der Praxis deshalb nicht verwendbar, weil sie viel zu groß, schwer, unhandlich und in der Herstellung zeitlich und preislich zu aufwendig ist.

So ist jeder einzelne Kanal etwa 40 cm lang, um die gewünschte Wirkung zu erzielen. Außerdem sind sogenannte "gaskets", d.h. Dichtungen + Abstandshalter, "supports", d.h. poröse Membranstützplatten, sowie eine massive Vorrichtung zum Zusammenpressen der Platten erforderlich. Viele Teile müssen einzeln hergestellt werden. Eine Plasmapherese-Vorrichtung ist jedoch zum Einmalgebrauch gedacht und muß deshalb wirtschaftlich realisierbar sein.

Aufgabe der Erfindung war es somit, eine Vorrichtung zur kontinuierlichen Plasmapherese auf Basis einer zu überströmenden Membran bereitzustellen, deren Kapazität die Anwendung bei der Plasmaspende gestattet und die sowohl von der Handhabung als auch vom wirtschaftlichen Standpunkt her realisierbar ist.

Bei der Suche nach einem solchen Modell versuchte man unter anderem, die sogenannte Strohmaier-Ultrafiltrations-Vorrichtung, die auf dem Überströmungsprinzip beruht, für die Plasmapherese einzusetzen. Diese Vorrichtung besteht aus übereinandergestapelten Platten, die Rillen einer bestimmten Breite mit einem dreieckigen Querschnitt besitzen, wobei sich die Rillen von jeweils zwei übereinandergeschichteten Platten kreuzen. Zwischen diese Platten wird eine Membran eingelegt. Diese Vorrichtung dient der Anreicherung und Dialyse hochmolekularer Stoffe, wie Eiweiß, Enzyme und Viren. Dabei stellte man fest, daß zwar bei Verwendung einer Membran der geeigneten Porengröße eine Abtrennung des Plasmas vom Blut möglich war, daß jedoch der Strömungswiderstand bei dieser Anordnung sehr hoch ist, so daß der Transmembrandruck derart ansteigt, daß unerwünschte Hämolyse auftritt.

Überraschenderweise wurde nun gefunden, daß eine auf dieser Ultrafiltrationsvorrichtung basierende Vorrichtung für die Plasmapherese Anwendung finden kann, wenn auf der sogenannten Blutseite ein ganz bestimmtes Verhältnis von Kanalbreite zu Kanaltiefe eingehalten wird und wenn die Blutkanäle über die Filtrationszone hinausreichen.

Die Erfindung betrifft eine Vorrichtung für die kontinuierliche Plasmapherese, gekennzeichnet durch mindestens eine Blutseite bestehend aus einer runden Platte (1) mit einem Blutzuflußkanal (3), einem Blutabflußkanal (4), Kanäle (5), die Blutzuflußkanal (3) und Blutabflußkanal (4) verbinden, und Kanalabgrenzungen (6), wobei die Breite der Kanäle das 4- bis 20-fache der Tiefe der Kanäle beträgt, und mindestens eine der Blutseite gegenüberliegende Plasmaseite, bestehend aus einer runden Platte (2) mit Plasmaabflußkanälen (4a) und/oder (4b), wobei die Dimensionen von Platte (2) und der Plasmaabflußkanäle (4a) bzw. (4b) im wesentlichen den Dimensionen der Platte (1) und dem Blutzuflußkanal (3) und dem Blutabflußkanal (4) entsprechen, den Kanälen (5a), die den Plasmaabflußkanal (4a) und den Plasmaabflußkanal (4b) verbinden, und den

Kanalbegrenzungen (6a), die nicht deckungsgleich mit dem Kanalmuster der Blutseite sind und so gestaltet sind, daß sie die dazwischenliegende Membran abzustützen vermögen, und daß durch den Zusammenbau der beiden Platten eine Filtrationszone gebildet wird, wobei die Kanäle (5) über die Filtrationszone hinausreichen.

Ein solches Gerät erfüllt in hervorragender Weise alle Bedingungen, die an eine kontinuierliche Plasmapherese im Zusammenhang mit der Plasmaspende gestellt werden und besitzt gegenüber dem in Abstracts (II) des XVII Congress of the Internatonal Society of Hematology, XV Congress of the International Society of Blood Transfusion vom 27. und 28. Juli 1978 beschriebenen Modell den Vorteil, daß es kleiner, leichter und handlicher und in der Herstellung einfach und billiger ist, daß keine separaten Membranträger und Dichtungen und keine Einspannvorrichtung benötigt werden, weil es aufgrund seiner Gestalt in sich dicht ist.

Die Kanäle auf der Blutseite müssen so breit sein, daß der Strömungswiderstand so gering wie möglich gehalten wird, dürfen andererseits jedoch nicht so breit sein, daß die Kanalgeometrie sich ändern kann, sei es durch Aufwölben der Membran oder durch Verformung der Platten. Je nach Art der Membran und des Materials der Platten beträgt die Kanalbreite das 4- bis 20-fache der Kanaltiefe, vorzugsweise das 8- bis 10-fache. Die Kanaltiefe kann bis zu 1 mm betragen vorzugsweise zwischen 0,1 und 0,5 mm, besonders bevorzugt beträgt sie 0,2 mm.

Durch dieses Breiten-Tiefen-Verhältnis erreicht man eine definierte Kanalgeometrie bei gleichzeitig geringem Druckabfall.

Diese Ausführung gewährleistet konstante Kanalgeometrie auch bei geringer Blutkanaltiefe, so daß bei einer praktikablen Strömungsgeschwindigkeit die Länge der Kanäle bei gleicher Filtrationsfläche reduziert werden kann.

Dadurch wird die Transmembran-Druckdifferenz verringert und eine divergierende Kanalgeometrie, wie sie in Abstracts (II) des XVII Congress of the International Society of Hematology, XV Congress of the International Society of Blood Transfusion vom 27. und 28. Juli 1978 beschrieben wird, erübrigt sich.

Die Blutkanäle reichen über die Filtrationszone hinaus, d.h. sie beginnen vor dem eigentlichen Filtrationsbereich, damit sich optimale Strömungsverhältnisse ausbilden können, bevor die Filtrationszone vom Blut erreicht wird. Unter Filtrationszone versteht man die Flächenbereiche, die unter den Blutkanälen auf der Plasmaseite den Plasmaabfluß gestatten.

Die Kanäle der Plasmaseite können analog der bekannten Ultrafiltrationsvorrichtung ausgebildet sein. Eine brauchbare Breite beträgt beispielsweise 0,8 mm. Sie können beispielsweise einen dreieckigen Querschnitt oder aber einen im wesentlichen rechteckigen Querschnitt aufweisen. Die Trennungsstege zwischen den Kanälen müssen nicht unbedingt zusammenhängend sein, sie können auch unterbrochen sein und als Noppen oder einem ähnlichen Muster ausgebildet sein. Wichtig ist lediglich, daß das Plasma zu den Plasmaabflußkanälen abfließen kann. Wichtig ist andererseits, daß das Muster der Plasmaseite so gestaltet ist,

daß es die Membran abzustützen vermag. Es soll deshalb nicht deckungsgleich sein mit der Blutseite, weil sonst die Plasmakanalbegrenzungen die Membran in die Blutkanäle eindrücken und somit die optimalen Strömungsverhältnisse stören können. Vorzugsweise sind die Plasmakanäle schmaler als die Blutkanäle und kreuzen diese.

Die runde Form der Vorrichtung bietet insbesondere herstellungstechnische Vorteile, d.h. sie ermöglicht eine einfache Herstellung durch Spritzgießen. Ein weiterer Vorteil besteht darin, daß handelsübliche Membranen eingesetzt werden können. Eine gängige Scheibennorm besitzt beispielsweise einen Durchmesser von 142 mm.

Obgleich für die Plasmapherese der Durchmesser der Vorrichtung nicht zwingend vorgeschrieben ist, sollte er aus den vorstehend genannten Gründen in dieser Größenordnung liegen.

Die Porengröße der einzusetzenden Membran soll in der für Membranplasmapherese bekannten Größenordnung liegen und beträgt etwa $0,2$ bis $0,8 \mu m$, vorzugsweise $0,5 \mu m$.

Obgleich es für die Funktion der Plasmapherese an sich keine Rolle spielt, aus welchem Material die Vorrichtung besteht, verwendet man zweckmäßigerweise, um die Vorteile der leichten und billigen Herstellung zu erzielen, ein Material, das sich durch Spritzguß verarbeiten läßt, wie beispielsweise Polystyrol und Polycarbonat.

Die Schaltung des Laufweges des Blutes und des abgetrennten Plasmas kann beliebig sein. Blutzufuhr, Ablauf der angereicherten Zellfraktion und Ablauf des Plasmas können seitlich an irgendeiner Stelle der Platten oder von oben oder unten erfolgen.

Auch die Anordnungsrichtung der einzelnen Platten ist beliebig. Sie können horizontal liegen, wobei es gleichgültig ist, ob die Blutseite oder die Plasmaseite nach oben zeigt. Sie können auch vertikal angeordnet sein. Um die Filtrationsfläche und damit die Kapazität zu vergrößern, können mehrere Einheiten übereinander angeordnet sein. Dabei können die innenliegenden Platten dünner ausgebildet sein.

Eine andere Ausführungsform besteht darin, daß die Außenplatten nur mit Schlauchanschlüssen versehen sind und erst die Innenplatten entsprechend mit Blut- und Plasmaseite(n) ausgestattet sind.

Besonders vorteilhaft ist es, wenn nur eine Außenplatte mit allen erforderlichen Anschlüssen ausgestattet ist. Bei Verwendung mehrerer Einheiten übereinander sind zweckmäßigerweise die Platten, die zwischen zwei aufeinanderfolgenden Membranen liegen, durch eine Platte ersetzt, die beidseitig mit Kanälen versehen ist. Dabei können entweder auf einer Seite Blutkanäle und auf der anderen Seite Plasmakanäle oder beidseitig Blut-/Blut- oder Plasma-/Plasma-Kanäle vorliegen. Fertigungstechnisch ist ersteres einfacher, weil man mit einer Spritzform für die Platten, die beidseitig mit Kanälen versehen sind, auskommt. Die Außenplatten sind so gebaut, daß sie den durch die Durchströmung entstandenen Druck ohne Verformung aufnehmen können. Eine ausreichende Stabilität gewährt eine Plattendicke von etwa 10 mm. Eine andere Möglichkeit besteht im Aufspritzen von Stegen auf die Außenplatten.

0021301

Die einzelnen Einheiten können entweder hintereinander, parallel oder in Kombination beider Anordnungen geschaltet sein.

Bei Verwendung mehrerer Platten können zweckmäßigerweise zwischen den einzelnen Platten auf der Plasmaseite Strömungswiderstände eingebaut werden,um die Transmembrandruckdifferenz zu verringern und somit eine eventuelle Hämolyse zu vermeiden.

Bei Verwendung nur eines Elements oder bei Parallelschaltung mehrerer Einheiten ist die Druckdifferenz ohnehin so gering, daß ein solcher Widerstand dabei nicht erforderlich ist.

Die Verbindung der Platten kann durch Verschrauben oder Nieten erfolgen, jedoch können die Platten auch zusammengedrückt und zusammen mit der eingelegten porösen Membran mit einem Kunststoffwulst umspritzt werden, der auch eine zusätzliche Abdichtung der Platten bewirkt.

Fig. 1 und 2 zeigen je eine Draufsicht auf eine zweckmäßige Ausführungsform einer Plasma- und einer Blutseite.

Fig. 3 zeigt schematisch eine mögliche Anordnung für die Platten einer erfindungsgemäßen Vorrichtung.

Fig. 4 und 5 zeigen schematisch die bei den nachstehend erläuterten Versuchen verwendeten Versuchsanordnungen.

Fig. 6 erläutert eine schematische Anordnung zur Plasmaentnahme, bei der kontinuierliche Plasmapherese mit Hilfe der erfindungsgemäßen Vorrichtung stattfindet.

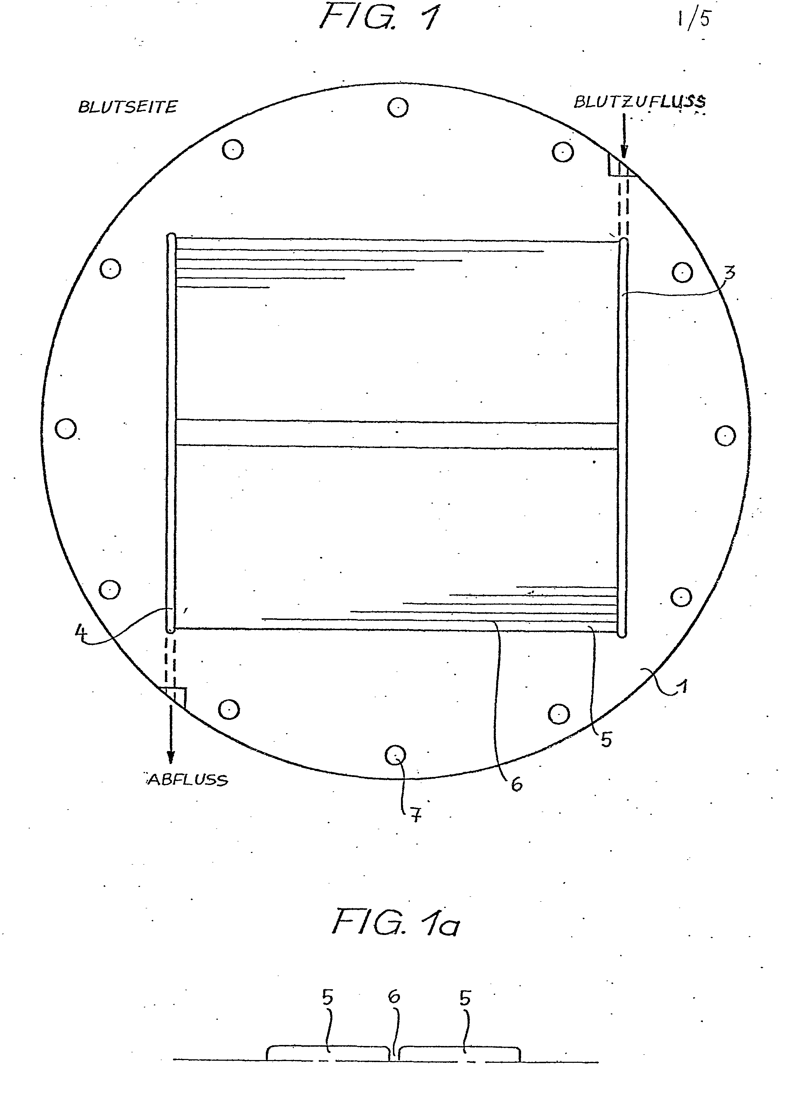

Fig. 1 zeigt die Blutseite, bestehend aus einer runden Platte (1), Blutzuflußkanal (3), Blutabflußkanal (4) und Kanäle (5) mit dazwischenliegenden Kanalbegrenzungen (6) in Form von Stegen. Die Platte (1) ist in dieser Ausführungsform um ihren Umfang mit Bohrungen (7) ausgestattet, durch die Schrauben bzw. Nieten zum Befestigen mit der oder den anderen Platte(n) hindurchgeführt werden können.

Fig. 1a zeigt den Querschnitt durch zwei Kanäle (5) und Steg (6), worin die Breite der Kanäle das 8-fache der Tiefe beträgt.

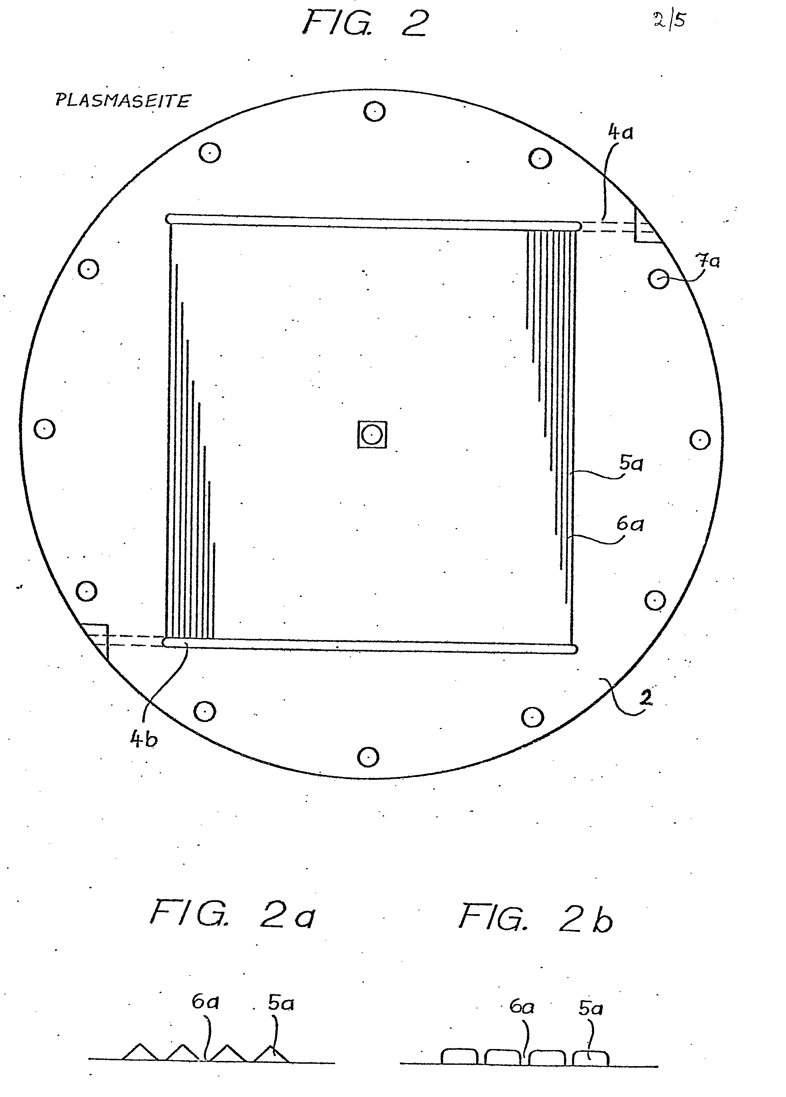

Fig. 2 zeigt die Plasmaseite, bestehend aus einer runden Platte (2) mit Plasmaabflußkanal (4a oder 4b), wobei einer der beiden Kanäle bei Gebrauch verschlossen wird. Die Kanäle (5a) mit dazwischenliegenden Kanalbegrenzungen (6a) in Form von Stegen sind so angeordnet, daß sie die Kanäle der Blutseite nach Zusammenbau kreuzen. Die Platte (2) ist in ihrem Umfang mit Bohrungen (7a) versehen, die sich in deckungsgleicher Position mit den Bohrungen (7) der Platte (1) befinden.

Fig. 2a zeigt einen Querschnitt durch mehrere Kanäle (5a) und Stege (6a) mit dreieckigen Kanälen und

Fig. 2b zeigt den gleichen Querschnitt durch mehrere Kanäle und Stege wie in Fig. 2a jedoch mit rechteckigen Kanälen.

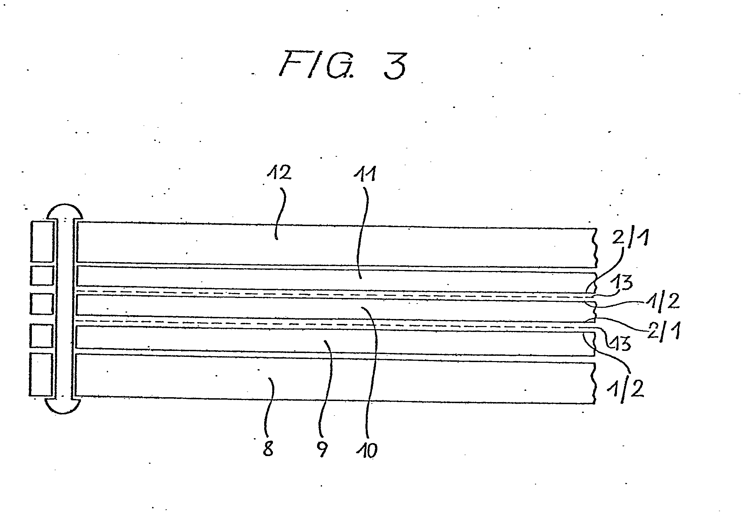

Fig. 3 zeigt schematisch eine mögliche Anordnung für die Platten einer erfindungsgemäßen Vorrichtung: Der Grundplatte (8) folgen die erste Innenplatte (9), die nur auf ihrer Oberseite als Plasma- (2) oder Blutseite (1) ausgebildet ist, die Innenplatte (10, die auf beiden Seiten entsprechend als Plasma- (2) oder Blutseite (1) ausgebildet ist, und die Innenplatte (11), die nur an ihrer Unterseite entsprechend als Plasma- (2) oder Blutseite (1) ausgebildet ist. Die Deckplatte (12) schließt die gesamte Anordnung ab. Zwischen den Kanälen befinden sich die porösen Membranen (13). Durch den weiteren Einbau von weiteren Innenplatten (10) läßt sich die Anzahl der Filtrationszonen vermehren, bis die gewünschte Leistung vorliegt.

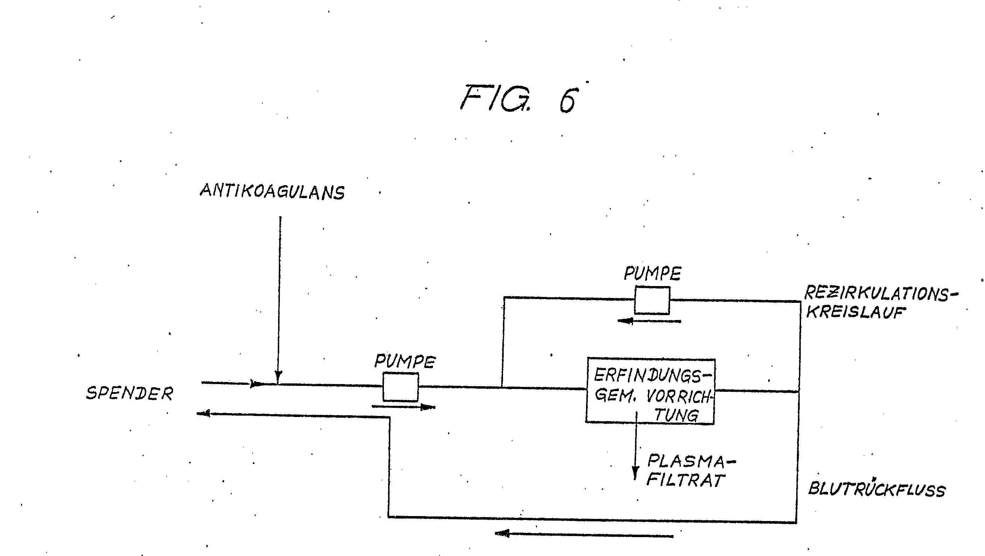

In Fig. 6 wird eine schematische Anordnung zur Plasmaentnahme bei der kont. Plasmapherese mit Hilfe der erfindungsgemäßen Vorrichtung gezeigt.
Das Blut des Spenders wird in einem bestimmten Volumenverhältnis mit Antikoagulans versetzt und durch eine Pumpe in den Rezirkulationskreislauf hineingepumpt. Der Rezirkulationskreislauf ist notwendig, damit das Blut in der Vorrichtung die notwendige Strömungsgeschwindigkeit aufweist. Plasma wird abgetrennt und das an Plasma verarmte Blut wird dem Spender wieder retransfundiert.

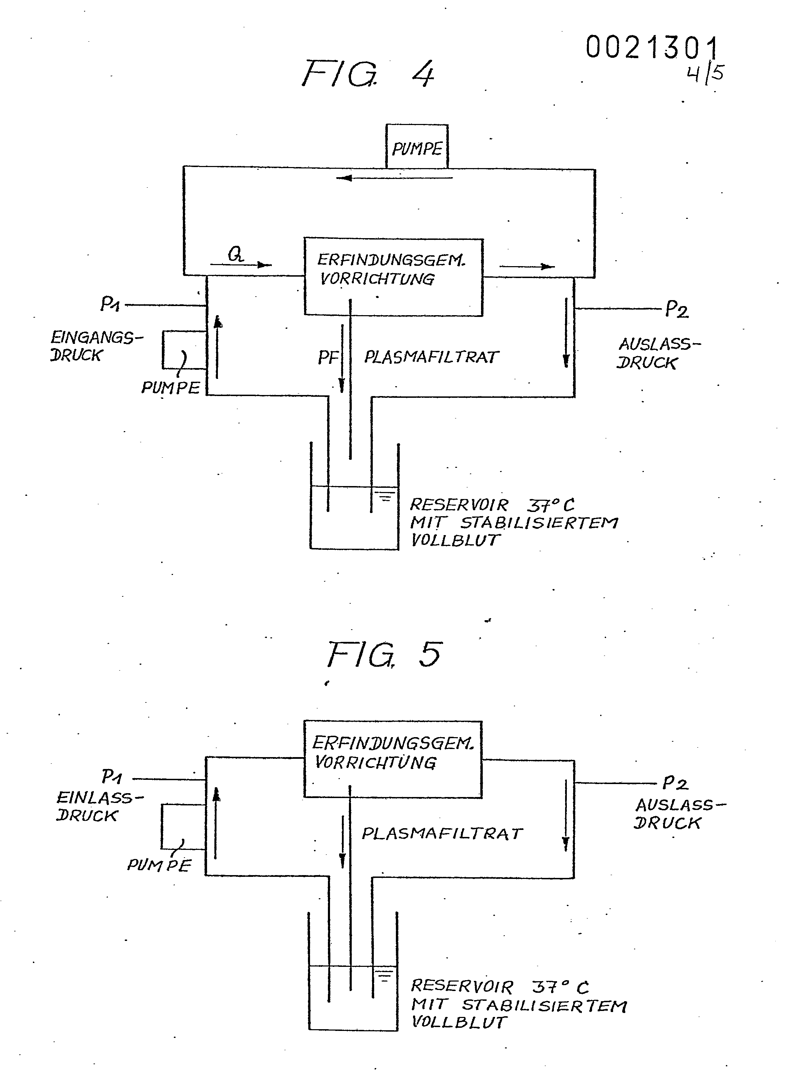

Nachstehende Versuche sollen zeigen, daß die Filtrationsleistung pro Flächeneinheit sowie die Hämolyse etwa dem der in Abstracts (II) des XVII Congress of the International Society of Hematology, XV Congress of the International Society of Blood Transfusion vom 27. und 28. Juli 1978 beschriebenen Vorrichtung entspricht.
Die Versuchsanordnung ist der Fig. 4 und 5 zu entnehmen.

Versuch Nr. 1

Vergleichsmodell

Versuchsanordnung gemäß Fig. 4 Filtrationsfläche: 400 cm²

Membranporengröße: 0,6 μm

Kanaldimension: Breite: von 1,1 cm auf 2,2 cm divergierend Tiefe: 0,375 mm Blutzuflußgeschwindigkeit: 72 ml/min.

Blutströmungsgeschwindigkeit am Eingang des Modelles:

140 ml/min.

Auslaßdruck: 79 Torr Hämatokrit des Blutes im Reservoir: 42 %

| Zeit | $\Delta p$ | PF | Hb mg/100 ml | | q |
|------|------|------|------|------|------|
| Min | Torr | ml/min | Plasmafiltrat | Blutplasma | ml/min·cm² |
| 0 | 130 | 16 | 33 | 31 | 0,040 |
| 1C | 126 | 16 | 61 | 37 | 0,040 |
| 20 | 128 | 14,5 | 70 | 49 | 0,036 |
| 30 | 124 | 12 | 73 | 58 | 0,030 |
| 40 | 124 | 10,2 | 74 | 66 | 0,026 |

$\Delta p$ Druckabfall $p_1-p_2$

PF Strömungsgeschwindigkeit des Plasmafiltrates

q Filtrationsleistung pro Filtrationsflächeneinheit

Versuch Nr. 2

erfindungsgemäße Vorrichtung aus 4 Einheiten, die parallel geschaltet sind.

Versuchsanordnung gemäß Fig. 4 Filtrationsfläche: 300 cm²

Membrangröße: 0,45 μm

Kanaldimension: Breite: 2,8 mm Tiefe: 0,35 mm Blutzuflußgeschwindigkeit: 60 ml/min.

Blutströmungsgeschwindigkeit am Eingang des Modelles:

siehe folgende Tabelle, Spalte Q Auslaßdruck: 100 Torr Hämatokrit des Blutes im Reservoir: 43 %

| Zeit min. | Q ml/min. | ΔP Torr | PF ml/min | Hb mg/100 ml Plasmafiltrat | Blutplasma | q ml/min·cm² |
|---|---|---|---|---|---|---|
| 0 | 510 | 70 | 12 | 40 | 26 | 0,040 |
| 10 | 610 | 84 | 11 | 40 | 34 | 0,037 |
| 20 | 685 | 108 | 15 | 57 | 43 | 0,050 |
| 30 | 685 | 110 | 15 | 67 | 55 | 0,050 |
| 40 | 685 | 112 | 15 | 77 | 67 | 0,050 |

Versuch Nr. 3

erfindungsgemäße Vorrichtung aus 2 Einheiten, die in Serie geschaltet sind.

Versuchsaufbau gemäß Fig. 5 Filtrationsfläche: 150 cm² Membranporengröße: 0,45 µm

Kanaldimension: Breite: 2,8 mm Tiefe: 0,35 mm Blutströmungsgeschwindigkeit am Eingang des Modelles:

138 ml/min Auslaßdruck: 104 Torr Hämatokrit des Blutes im Reservoir: ~40 %.

| Zeit | $\Delta$p | PF | Hb mg/100 ml | | q |
|------|-----------|----|--------------|------------|------------|
| min. | Torr | ml/min. | Plasmafiltrat | Blutplasma | ml/min.cm² |
| 0 | 82 | 8,2 | 13 | 5 | 0,055 |
| 10 | 80 | 8,4 | 24 | 9 | 0,056 |
| 20 | 80 | 8,6 | 26 | 16 | 0,057 |
| 30 | 80 | 8,1 | 31 | 21 | 0,054 |
| 40 | 82 | 7,0 | 33 | 29 | 0,047 |

0021301

Versuch Nr. 4

erfindungsgemäße Vorrichtung aus 1 Einheit Versuchsanordnung gemäß Fig. 5 Filtrationsfläche: 75 cm²

Membranporengröße: 0,45 μm

Kanaldimension: Breite: 0,9 mm Tiefe: 0,1 mm Blutströmungsgeschwindigkeit am Eingang des Modelles:

13 ml/min.

Auslaßdruck: 100 Torr Hämatokrit des Blutes im Reservoir: 45 %

| Zeit | $\Delta$p | PF | Hb mg/100 ml | | q |
|------|-----------|------|--------------|--------------|------------|
| min | Torr | ml/min. | Plasmafiltrat | Blutplasma | ml/min.cm² |
| 0 | 62 | 2,7 | 28 | 19 | 0,036 |
| 10 | 60 | 2,3 | 29 | 22 | 0,031 |
| 20 | 56 | 2,1 | 28 | 22 | 0,028 |
| 30 | 72 | 2,0 | 32 | 28 | 0,027 |
| 40 | 62 | 2,0 | 33 | 26 | 0,027 |

0021301

Versuch Nr. 5

erfindungsgemäße Vorrichtung aus 1 Einheit Versuchsanordnung gemäß Fig. 5 Filtrationsfläche:   75 cm² Membranporengröße: 0,45 µm Kanaldimension:  Breite:   1,8 mm Tiefe:   0,2 mm Blutströmungsgeschwindigkeit am Eingang des Modelles:

70 ml/min.

Auslaßdruck:   100 Torr Hämatokrit des Blutes im Reservoir:   54 %

| Zeit min. | $\Delta$p Torr | PF ml/min. | Hb mg/100 ml Plasmafiltrat | Blutplasma | q ml/min.cm² |
|---|---|---|---|---|---|
| 0 | 74 | 4,2 | 16 | 8 | 0,056 |
| 10 | 70 | 4,1 | 17 | 12 | 0,055 |
| 20 | 74 | 4,5 | 31 | 18 | 0,060 |
| 30 | 76 | 4,5 | 31 | 25 | 0,060 |
| 40 | 76 | 4,4 | 34 | 31 | 0.059 |

Nachstehender Versuch soll demonstrieren, daß eine Plasmapherese ohne die unerwünschte Hämolyse nur mit der erfindungsgemäß beanspruchten Kanaldimension möglich ist.

Versuch Nr. 6

Satorius-Ultrafiltrationsvorrichtung nach Strohmeier mit 1 Membran Versuchsan ordnung gemäß Fig. 5 Filtrationsfläche: 160 cm² Membranporengröße: 0,45 µm Blutströmungsgeschwindigkeit am Eingang des Modelles:

53 ml/min Auslaßdruck: 36 Torr Hämatokrit des Blutes im Reservoir: 38 %

| Zeit min. | $\Delta p$ Torr | PF ml/min. | Hb mg/100 ml | | q ml/min.cm² |
|-----------|-----------------|------------|--------------|--|---------------|
| | | | Plasmafiltrat | Blutplasma | |
| 0 | 126 | 6,2 | 71,3 | 18,6 | 0,039 |
| 10 | 146 | 7,0 | 50,4 | 24,8 | 0,044 |
| 20 | 156 | 7,0 | 45,7 | 31,8 | 0,044 |
| 30 | 156 | 6,7 | 41,1 | 34,9 | 0,042 |

Patentansprüche

1. Vorrichtung für die kontinuierliche Plasmapherese, gekennzeichnet durch mindestens eine Blutseite, bestehend aus einer runden Platte (1) mit einem Blutzuflußkanal (3), einem Blutabflußkanal (4), Kanäle (5), die Blutzuflußkanal (3) und Blutabflußkanal (4) verbinden, und Kanalabgrenzungen (6), wobei die Breite der Kanäle das 4- bis 20-fache der Tiefe der Kanäle beträgt,
und mindestens eine der Blutseite gegenüberliegende Plasmaseite, bestehend aus einer runden Platte (2) mit Plasmaabflußkanälen (4a) und/oder (4b), wobei die Dimensionen von Platte (2) und der Plasmaabflußkanäle (4a) bzw. (4b) im wesentlichen den Dimensionen der Platte (1) und dem Blutzuflußkanal (3) und dem Blutabflußkanal (4) entsprechen, den Kanälen (5a), die den Plasmaabflußkanal (4a) und den Plasmaabflußkanal (4b) verbinden, und den Kanalbegrenzungen (6a), die nicht deckungsgleich mit dem Kanalmuster der Blutseite sind und so gestaltet sind, daß sie die dazwischenliegende Membran abzustützen vermögen,
und daß durch den Zusammenbau der beiden Platten eine Filtrationszone gebildet wird, wobei die Kanäle (5) über die Filtrationszone hinausreichen.

2. Vorrichtung nach Anspruch 1, dadurch gekennzeichnet, daß die Plasmaseite Kanäle (5a) aufweist, die

0021301

schmaler sind als die Kanäle (5) der Blutseite und dieselben kreuzen.

3. Vorrichung nach Anspruch 1 oder 2, dadurch gekennzeichnet, daß die die Blutseite tragende Platte (1) und die die Plasmaseite tragende Platte (2) mit einer dazwischenliegenden mikroporösen Membran fest miteinander verbunden sind.

4. Vorrichtung nach Anspruch 3, dadurch gekennzeichnet, daß die Platten durch Umspritzen miteinander verbunden sind.

5. Vorrichtung nach einem der vorstehenden Ansprüche, dadurch gekennzeichnet, daß mehrere Einheiten übereinander angeordnet und die innenliegenden Platten dünner ausgebildet sind.

6. Vorrichtung nach einem der vorstehenden Ansprüche, dadurch gekennzeichnet, daß mehrere eine Blut- und eine Plasmaseite aufweisende Einheiten hintereinander, parallel oder in Kombination beider Anordnungen geschaltet sind.

7. Vorrichtung nach Anspruch 5, dadurch gekennzeichnet, daß sich zwischen den Platten auf der Plasmaseite Strömungswiderstände zur Verringerung der Transmembrandruckdifferenz befinden.

8. Vorrichtung nach Anspruch 5, dadurch gekennzeichnet, daß die innenliegenden Platten beidseitig mit Kanälen versehen sind, die entweder beidseitig Blut bzw. Plasma oder auf der einen Seite Blut und auf der Gegenseite Plasma führen.

FIG. 1

BLUTSEITE

BLUTZUFLUSS

3

4

ABFLUSS

1

5

6

7

FIG. 1a

5    6    5

# FIG. 2

PLASMASEITE

4a

7a

5a

6a

2

4b

# FIG. 2a

6a  5a

# FIG. 2b

6a  5a

*FIG. 3*

# FIG. 4

PUMPE

Q →

ERFINDUNGSGEM. VORRICHTUNG

$P_1$ — EINGANGS-DRUCK

PUMPE

PF PLASMAFILTRAT

$P_2$ — AUSLASS-DRUCK

RESERVOIR 37°C MIT STABILISIERTEM VOLLBLUT

# FIG. 5

ERFINDUNGSGEM. VORRICHTUNG

$P_1$ — EINLASS-DRUCK

PUMPE

PLASMAFILTRAT

$P_2$ AUSLASS-DRUCK

RESERVOIR 37°C MIT STABILISIERTEM VOLLBLUT

## FIG. 6

ANTIKOAGULANS

SPENDER

PUMPE

PUMPE

REZIRKULATIONS-
KREISLAUF

ERFINDUNGS-
GEM. VORRICH-
TUNG

PLASMA-
FILTRAT

BLUTRÜCKFLUSS

0021301
5/5